# EUROPÄISCHE PATENTSCHRIFT

(11) **EP 0 659 746 B1**
(45) Veröffentlichungstag und Bekanntmachung des Hinweises auf die Patenterteilung: **06.05.1998**
(21) Anmeldenummer: 94119400.3
(22) Anmeldetag: 08.12.1994
(51) Int. Cl.: C07D 249/12

(54) **Verfahren zur Herstellung von Sulfonylaminocarbonyltriazolinonen**
Process for the preparation of sulfonylaminocarbonyl-triazolinones
Procédé pour la préparation de sulfonyaminocarbonyl-triazolinones

(30) Priorität: 21.12.1993 DE 4343595
(43) Veröffentlichungstag der Anmeldung: 28.06.1995
(73) Patentinhaber: BAYER AG, 51368 Leverkusen (DE)
(72) Erfinder: Kluth, Joachim, Dr., D-40764 Langenfeld (DE); Müller, Klaus-Helmut, Dr., D-40593 Düsseldorf (DE)

(56) Entgegenhaltungen:
- EP-A- 0 422 469
- EP-A- 0 537 585
- GB-A- 2 150 139
- Journal f. prakt. Chemie, Band 330, Heft 5, 1988, S.753-758

## Beschreibung

Die Erfindung betrifft ein neues Verfahren zur Herstellung von Sulfonylaminocarbonyltriazolinonen, welche als Herbizide verwendet werden können.

Es ist bekannt, daß man Sulfonylaminocarbonyltriazolinone erhält, wenn man Triazolinone mit Sulfonylisocyanaten umsetzt (vgl. EP-A 341489, EP-A 422469, EP-A 425948, EP-A 431291, EP-A 507171, EP-A 534266).

Die bei diesem bekannten Verfahren als Ausgangsstoffe benötigten Sulfonylisocyanate müssen jedoch in einem vorausgehenden Verfahrensschritt - im allgemeinen aus entsprechenden Sulfonamiden und Phosgen - hergestellt werden.

Weiterhin ist bekannt, daß man bestimmte substituierte N-Phenylsulfonyl-N'-(pyrimidin-2-yl)- und -N'-(1,3,5-triazin-2-yl)-harnstoffe erhält, wenn man entsprechende Phenyl-sulfochloride mit einem Alkalimetallcyanat und 2-Amino-pyrimidinen bzw. 2-Amino-1,3,5-triazinen umsetzt, wobei die organischen Ausgangsstoffe so ausgewählt werden müssen, daß die einfache Sulfonamidbildung aus dem Sulfochlorid und der Amino-Verbindung - d.h. ohne Einschiebung der aus dem Metallcyanat stammenden Aminocarbonyl-Einheit 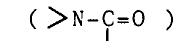 - als unerwünschte Nebenreaktion weitgehend vermieden wird (vgl. GB-A 2 150 139).

Außerdem ist bekannt, daß bestimmte N-(2,6-disubstituierte Phenylsulfonyl)-N'-(pyrimidin-2-yl)- und -N'(1,3,5-triazin-2-yl)-harnstoffe besonders günstig durch basen-katalysierte Umsetzung der entsprechenden Phenyl-sulfochloride mit einem Alkalimetallcyanat und 2-Amino-pyrimidinen bzw. 2-Amino-1,3,5-triazinen in Gegenwart eines aprotischen Lösungsmittels erhalten werden können (vgl. EP-A 537 585).

Es wurde nun gefunden, daß man Sulfonylaminocarbonyltriazolinone der allgemeinen Formel (I) 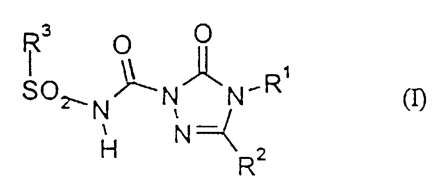 in welcher
- R¹: für einen gegebenenfalls substituierten Rest aus der Reihe Alkyl, Alkenyl, Alkinyl, Alkoxy, Alkenyloxy, Dialkylamino, Cycloalkyl, Cycloalkyloxy, Cycloalkylalkyl, Aryl, Arylalkyl steht,
- R²: für Wasserstoff, Halogen oder für einen gegebenenfalls substituierten Rest aus der Reihe Alkyl, Alkenyl, Alkinyl, Alkoxy, Alkenyloxy, Alkinyloxy, Alkylthio, Alkenylthio, Alkinylthio, Alkylsulfinyl, Alkylsulfonyl, Dialkylamino, Cycloalkyl, Cycloalkenyl, Cycloalkyloxy, Cycloalkenyloxy, Cycloalkylthio, Cycloalkenylthio, Cycloalkylalkyl, Cycloalkenylalkyl, Aryl, Aryloxy, Arylthio, Arylalkyl, Arylalkyloxy, Arylalkylthio steht, und
- R³: für einen gegebenenfalls substituierten Rest aus der Reihe Alkyl, Cycloalkyl, Cycloalkylalkyl, Aryl, Arylalkyl, Heterocyclyl, Heterocyclylalkyl steht,
in guten Ausbeuten und in hoher Reinheit erhält, wenn man Triazolinone der allgemeinen Formel (II) 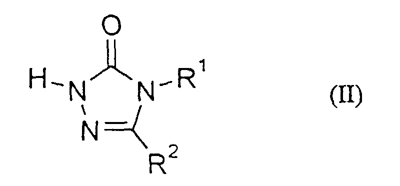 in welcher
- R¹ und R²: die oben angegebene Bedeutung haben,
mit Sulfonsäurehalogeniden der allgemeinen Formel (III)

R³-SO₂-X (III)

in welcher
- R³: die oben angegebene Bedeutung hat und
- X: für Halogen steht,
und Metallcyanaten der allgemeinen Formel (IV)

MOCN (IV)

in welcher
- M: für ein Alkalimetall oder ein Erdalkalimetall-äquivalent steht,
gegebenenfalls in Gegenwart eines Reaktionshilfsmittels und gegebenenfalls in Gegenwart eines Verdünnungsmittels bei Temperaturen zwischen 20°C und 150°C umsetzt.

Es ist als überraschend anzusehen, daß das erfindungsgemäße Verfahren als eine gleichzeitige Umsetzung dreier verschiedener Komponenten in glatter Reaktion die Sulfonylaminotriazolinone der Formel (I) in guten Ausbeuten und in hoher Reinheit ergibt.

Als eine sehr vereinfachte Herstellungsmethode für die Verbindungen der Formel (I) stellt das erfindungsgemäße Verfahren eine wertvolle Bereicherung des Standes der Technik dar.

Das erfindungsgemäße Verfahren betrifft vorzugsweise die Herstellung von Sulfonylaminotriazolinonen der Formel (I), in welcher
- R¹: für gegebenenfalls durch Halogen, Cyano, C₁-C₄-Alkoxy, C₁-C₄-Alkyl-carbonyl oder C₁-C₄-Alkoxy-carbonyl substituiertes Alkyl mit 1 bis 6 Kohlenstoffatomen, für jeweils gegebenenfalls durch Halogen substituiertes Alkenyl oder Alkinyl mit jeweils 2 bis 6 Kohlenstoffatomen, für jeweils gegebenenfalls durch Halogen substituiertes Alkoxy, Alkenyloxy oder Dialkylamino mit jeweils bis zu 5 Kohlenstoffatomen in den Alkyl- bzw. Alkenyl-gruppen, für jeweils gegebenenfalls durch Halogen, Cyano und/oder C₁-C₄-Alkyl substituiertes Cycloalkyl, Cycloalkyloxy oder Cycloalkylalkyl mit jeweils 3 bis 6 Kohlenstoffatomen in den Cycloalkylgruppen und gegebenenfalls 1 bis 4 Kohlenstoffatomen im Alkylteil, oder für jeweils gegebenenfalls durch Halogen, Cyano, Nitro, C₁-C₄-Alkyl, C₁-C₄-Halogenalkyl, C₁-C₄-Alkoxy und/oder C₁-C₄-Halogenalkoxy substituiertes Phenyl, Naphthyl, Phenylalkyl oder Naphthylalkyl mit 1 bis 4 Kohlenstoffatomen in den Alkylteilen steht,
- R²: für Wasserstoff, Halogen, für gegebenenfalls durch Halogen, Cyano, C₁-C₄-Alkoxy, C₁-C₄-Alkyl-carbonyl oder C₁-C₄-Alkoxy-carbonyl substituiertes Alkyl mit 1 bis 6 Kohlenstoffatomen, für jeweils gegebenenfalls durch Halogen substituiertes Alkenyl oder Alkinyl mit jeweils 2 bis 6 Kohlenstoffatomen, für jeweils gegebenenfalls durch Halogen substituiertes Alkoxy, Alkenyloxy, Alkinyloxy, Alkylthio, Alkenylthio, Alkinylthio, Alkylsulfinyl, Alkylsulfonyl, Dialkylamino mit jeweils bis zu 5 Kohlenstoffatomen, für jeweils gegebenenfalls durch Halogen, Cyano oder C₁-C₄-Alkyl substituiertes Cycloalkyl, Cycloalkenyl, Cycloalkyloxy, Cycloalkenyloxy, Cycloalkylthio, Cycloalkenylthio, Cycloalkylalkyl oder Cycloalkenylalkyl mit jeweils 3 bis 6 Kohlenstoffatomen in den Cycloalkylgruppen und gegebenenfalls 1 bis 4 Kohlenstoffatomen in den Alkylgruppen, für jeweils gegebenenfalls durch Halogen, Cyano, Nitro, C₁-C₄-Alkyl, C₁-C₄-Halogenalkyl, C₁-C₄-Alkoxy und/oder C₁-C₄-Halogenalkoxy substituiertes Phenyl, Naphthyl, Phenoxy, Naphthyloxy, Phenylthio, Naphthylthio, Phenylmethyl, Phenylethyl, Phenylmethoxy, Naphthylmethoxy, Phenylethoxy, Naphthylethoxy, Phenylmethylthio, Naphthylmethylthio, Phenylethylthio oder Naphthylethylthio steht, und
- R³: für gegebenenfalls durch Halogen, Cyano, C₁-C₄-Alkoxy, C₁-C₄-Alkyl-carbonyl oder C₁-C₄-Alkoxy-carbonyl substituiertes Alkyl mit 1 bis 6 Kohlenstoffatomen, für jeweils gegebenenfalls durch Halogen, Cyano, Carboxy, C₁-C₄-Alkyl und/oder C₁-C₄-Alkoxy-carbonyl substituiertes Cycloalkyl oder Cycloalkylalkyl mit 3 bis 6 Kohlenstoffatomen in den Cycloalkylgruppen und gegebenenfalls 1 bis 4 Kohlenstoffatomen im Alkylteil, oder für jeweils gegebenenfalls durch Halogen, Cyano, Carboxy, Nitro, C₁-C₄-Alkyl, C₁-C₄-Halogenalkyl, C₁-C₄-Alkoxy-C₁-C₄-alkyl, C₁-C₄-Alkoxy, C₁-C₄-Halogenalkoxy, C₁-C₄-Alkoxy-C₁-C₄-alkoxy, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl, Di-(C₁-C₃-alkyl)-amino-sulfonyl, N-(C₁-C₃-Alkoxy)-N-(C₁-C₃-alkyl)-amino-sulfonyl, C₁-C₄-Alkyl-carbonyl, C₁-C₄-Alkoxy-carbonyl, C₁-C₄-Halogenalkoxy-carbonyl, Di-(C₁-C₃-alkyl)-amino-carbonyl, C₁-C₂-Alkoxy-C₁-C₂-alkoxy-carbonyl, C₃-C₆-Cycloalkyl, C₃-C₆-Cycloalkyl-methyl, C₃-C₆-Cycloalkyl-carbonyl, Phenyl oder Phenoxy substituiertes Phenyl, Naphthyl, Benzyl, Phenylethyl, Pyridyl, Pyridylmethyl, Pyridylethyl, Chinolyl, Chinolylmethyl, Thienyl, Thienylmethyl, Pyrazolyl oder Pyrazolylmethyl steht.

Die bei den Restedefinitionen genannten Kohlenwasserstoffreste, wie Alkyl, Alkenyl oder Alkinyl, auch in Kombinationen mit Heteroatomen, wie in Alkoxy, Alkylthio oder Alkylamino, sind auch dann, wenn dies nicht ausdrücklich angegeben ist, geradkettig oder verzweigt.

Halogen steht im allgemeinen für Fluor, Chlor, Brom oder Iod, vorzugsweise für Fluor, Chlor oder Brom, insbesondere für Fluor oder Chlor.

Das erfindungsgemäße Verfahren betrifft insbesondere die Herstellung von Verbindungen der Formel (I), in welcher
- R¹: für jeweils gegebenenfalls durch Fluor, Chlor, Brom, Cyano, Methoxy, Ethoxy, Acetyl, Propionyl, Methoxycarbonyl oder Ethoxycarbonyl substituiertes Methyl, Ethyl, n- oder i-Propyl, n-, i-, s- oder t-Butyl, für jeweils gegebenenfalls durch Fluor, Chlor und/oder Brom substituiertes Propenyl, Butenyl, Propinyl oder Butinyl, für jeweils gegebenenfalls durch Fluor, Chlor und/oder Brom substituiertes Methoxy, Ethoxy, n- oder i-Propoxy, n-, i-, s- oder t-Butoxy, Propenyloxy, Butenyloxy, Dimethylamino oder Diethylamino, für jeweils gegebenenfalls durch Fluor, Chlor, Brom, Cyano, Methyl, Ethyl, n- oder i-Propyl substituiertes Cyclopropyl, Cyclobutyl, Cyclopentyl, Cyclohexyl, Cyclopropyloxy, Cyclobutyloxy, Cyclopentyloxy, Cyclohexyloxy, Cyclopropylmethyl, Cyclopropylethyl, Cyclobutylmethyl, Cyclobutylethyl, Cyclopentylmethyl, Cyclopentylethyl, Cyclohexylmethyl oder Cyclohexylethyl, oder für jeweils gegebenenfalls durch Fluor, Chlor, Brom, Cyano, Nitro, Methyl, Ethyl, n- oder i-Propyl, Trifluormethyl, Methoxy, Ethoxy, Difluormethoxy und/oder Trifluormethoxy substituiertes Phenyl, Benzyl oder Phenylethyl steht,
- R²: für Wasserstoff, Fluor, Chlor, Brom, für jeweils gegebenenfalls durch Fluor, Chlor, Brom, Cyano, Methoxy, Ethoxy, Acetyl, Propionyl, Methoxycarbonyl oder Ethoxycarbonyl substituiertes Methyl, Ethyl, n- oder i-Propyl, n-, i-, s- oder t-Butyl, für jeweils gegebenenfalls durch Fluor, Chlor und/oder Brom substituiertes Propenyl, Butenyl, Propinyl oder Butinyl, für jeweils gegebenenfalls durch Fluor, Chlor und/oder Brom substituiertes Methoxy, Ethoxy, n- oder i-Propoxy, n-, i-, s- oder t-Butoxy, Propenyloxy, Butenyloxy, Propinyloxy, Butinyloxy, Methylthio, Ethylthio, n- oder i-Propylthio, n-, i-, s- oder t-Butylthio, Propenylthio, Butenylthio, Propinylthio, Butinylthio, Methylsulfinyl, Ethylsulfinyl, Propylsulfinyl, Methylsulfonyl, Ethylsulfonyl, Propylsulfonyl, Dimethylamino oder Diethylamino, für jeweils gegebenenfalls durch Fluor, Chlor, Brom, Cyano, Methyl, Ethyl, n- oder i-Propyl substituiertes Cyclopropyl, Cyclobutyl, Cyclopentyl, Cyclohexyl, Cyclopentenyl, Cyclohexenyl, Cyclopropyloxy, Cyclobutyloxy, Cyclopentyloxy, Cyclohexyloxy, Cyclopentenyloxy, Cyclohexenyloxy, Cyclopropylthio, Cyclobutylthio, Cyclopentylthio, Cyclohexylthio, Cyclopentenylthio, Cyclohexenylthio, Cyclopropylmethyl, Cyclopropylethyl, Cyclobutylmethyl, Cyclobutylethyl, Cyclopentylmethyl, Cyclopentylethyl, Cyclohexylmethyl, Cyclohexylethyl, Cyclopentenylmethyl oder Cyclohexenylmethyl, für jeweils gegebenenfalls durch Fluor, Chlor, Brom, Cyano, Nitro, Methyl, Ethyl, n- oder i-Propyl, Trifluormethyl, Methoxy, Ethoxy, Difluormethoxy und/oder Trifluormethoxy substituiertes Phenyl, Phenoxy, Phenylthio, Phenylmethyl, Phenylmethoxy oder Phenylmethylthio steht, und
- R³: für gegebenenfalls durch Fluor, Chlor, Brom, Cyano, Methoxy, Ethoxy, n-oder i-Propoxy, Acetyl, Propionyl, Methoxycarbonyl oder Ethoxycarbonyl substituiertes Methyl, Ethyl, n- oder i-Propyl, n-, i-, s- oder t-Butyl, für jeweils gegebenenfalls durch Fluor, Chlor, Brom, Cyano, Carboxy, Methyl, Ethyl, n- oder i-Propyl, Methoxycarbonyl und/oder Ethoxycarbonyl substituiertes Cyclopropyl, Cyclobutyl, Cyclopentyl, Cyclohexyl, Cyclopropylmethyl, Cyclopropylethyl, Cyclobutylmethyl, Cyclobutylethyl, Cyclopentylmethyl, Cyclopentylethyl, Cyclohexylmethyl oder Cyclohexylethyl, oder für jeweils gegebenenfalls durch Fluor, Chlor, Brom, Cyano, Carboxy, Nitro, Methyl, Ethyl, n- oder i-Propyl, Fluormethyl, Chlormethyl, Difluormethyl, Dichlormethyl, Trifluormethyl, Trichlormethyl, Fluorethyl, Chlorethyl, Difluorethyl, Dichlorethyl, Trifluorethyl, Tetrafluorethyl, Pentafluorethyl, Fluorpropyl, Chlorpropyl, Difluorpropyl, Dichlorpropyl, Trifluorpropyl, Trichlorpropyl, Methoxymethyl, Ethoxymethyl, Methoxyethyl, Ethoxyethyl, Methoxy, Ethoxy, n- oder i-Propoxy, Difluormethoxy, Trifluormethoxy, Difluorethoxy, Trifluorethoxy, Methoxyethoxy, Ethoxy-ethoxy, Methylthio, Ethylthio, n- oder i-Propylthio, Difluormethylthio, Trifluormethylthio, Methylsulfinyl, Ethylsulfinyl, Propylsulfinyl, Trifluormethylsulfinyl, Methylsulfonyl, Ethylsulfonyl, Propylsulfonyl, Trifluormethylsulfonyl, Dimethylaminosulfonyl, Diethylaminosulfonyl, N-Methoxy-N-methyl-amino-sulfonyl, Acetyl, Propionyl, Methoxycarbonyl, Ethoxycarbonyl, Propoxycarbonyl, Fluorethoxycarbonyl, Chlorethoxycarbonyl, Dimethylaminocarbonyl, Diethylaminocarbonyl, Methoxyethoxycarbonyl, Ethoxyethoxycarbonyl, Cyclopropyl, Cyclobutyl, Cyclopentyl, Cyclohexyl, Cyclopropylmethyl, Cyclobutylmethyl, Cyclopentylmethyl, Cyclopropylcarbonyl, Cyclobutylcarbonyl, Cyclopentylcarbonyl, Cyclohexylcarbonyl, Phenyl oder Phenoxy substituiertes Phenyl, Naphthyl, Benzyl, Phenylethyl, Pyridyl, Pyridylmethyl, Pyridylethyl, Chinolyl, Chinolylmethyl, Thienyl, Thienylmethyl, Pyrazolyl oder Pyrazolylmethyl steht.

Die oben aufgeführten allgemeinen oder in Vorzugsbereichen angegebenen Restedefinitionen gelten sowohl für die Endprodukte der Formel (I) als auch entsprechend für die zu Herstellung benötigten Ausgangsstoffe.

Diese Restedefinitionen können untereinander, also auch zwischen den angegebenen Bereichen bevorzugter Verbindungen, beliebig kombiniert werden.

Verwendet man beispielsweise 2-Fluor-benzolsulfonsäurechlorid und 4-Methoxy-5-methyl-2,4-dihydro-3H-1,2,4-triazol-3-on sowie Kaliumcyanat als Ausgangsstoffe, so kann der Reaktionsablauf beim erfindungsgemäßen Verfahren durch das folgende Formelschema skizziert werden:

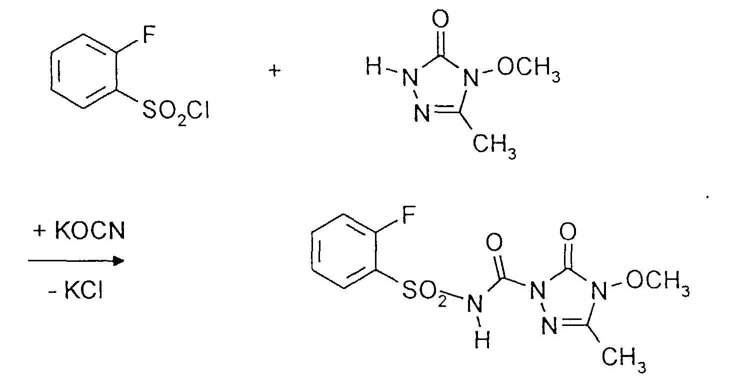

Die beim erfindungsgemäßen Verfahren zur Herstellung der Verbindungen der allgemeinen Formel (I) als Ausgangsstoffe zu verwendenden Triazolinone sind durch die Formel (II) allgemein definiert. In der Formel (II) haben R¹ und R² vorzugsweise bzw. insbesondere diejenigen Bedeutungen, die bereits oben im Zusammenhang mit der Beschreibung der erfindungsgemäß herzustellenden Verbindungen der Formel (I) als bevorzugt bzw. als insbesondere bevorzugt für R¹ und R² angegeben wurden.

Die Triazolinone der allgemeinen Formel (II) sind bekannt und/oder können nach an sich bekannten Verfahren hergestellt werden (vgl. EP-A 341489, EP-A 403889, EP-A 422469, EP-A 425948, EP-A 431291, EP-A 507171, EP-A 534266).

Die beim erfindungsgemäßen Verfahren weiter als Ausgangsstoffe zu verwendenden Sulfonsäurehalogenide sind durch die Formel (III) allgemein definiert. In der Formel (III) hat R³ vorzugsweise bzw. insbesondere diejenige Bedeutung, die bereits oben im Zusammenhang mit der Beschreibung der Verbindungen der Formel (I) als bevorzugt bzw. als insbesondere bevorzugt für R³ angegeben wurde; X steht vorzugsweise für Fluor, Chlor oder Brom, insbesondere für Chlor.

Die Sulfonsäurehalogenide der Formel (III) sind bekannt und/oder können nach an sich bekannten Verfahren hergestellt werden (vgl. J. Org. Chem. 25 (1960), 1824; loc. cit. 33 (1968), 2104; EP-A 23140; EP-A 23141; EP-A 23422; EP-A 35893; EP-A 44808; EP-A 44809; EP-A 48143; EP-A 51466; EP-A 64322; EP-A 70041).

Die beim erfindungsgemäßen Verfahren weiter als Ausgangsstoffe zu verwendenden Metallcyanate sind durch die Formel (IV) allgemein definiert. In der Formel (IV) steht M vorzugsweise für Lithium, Natrium, Kalium, ein Magnesium- oder ein Calciumäquivalent, insbesondere für Natrium oder Kalium.

Die Ausgangsstoffe der Formel (IV) sind bekannte Synthesechemikalien.

Das erfindungsgemäße Verfahren wird gegebenenfalls in Gegenwart eines geeigneten Reaktionshilfsmittels durchgeführt. Als solche kommen vorzugsweise organische Stickstoffbasen in Betracht. Hierzu gehören insbesondere tertiäre Amine, wie Trimethylamin, Triethylamin, Tripropylamin, Tributylamin, N,N-Dimethylanilin, N,N-Dimethylbenzylamin, sowie Stickstoffheterocyclen, wie Pyridin, Methylpyridine, N-Methylpiperidin, N,N-Dimethylaminopyridin, Diazabicyclooctan (DABCO), Diazabicyclononen (DBN) oder Diazabicycloundecen (DBU).

Das erfindungsgemäße Verfahren wird vorzugsweise in Gegenwart eines Verdünnungsmittels durchgeführt. Als Verdünnungsmittel zur Durchführung des erfindungsgemäßen Verfahrens kommen vor allem inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlormethan; Ether, wie Diethylether, Diisopropylether, Dioxan, Tetrahydrofuran oder Ethylenglykoldimethyl- oder -diethylether; Ketone, wie Aceton, Butanon, Methyl-isopropylketon oder Methyl-isobutylketon; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester, sowie Sulfoxide, wie Dimethylsulfoxid.

Hiervon werden die aprotisch polaren Solventien, wie Aceton, Butanon, Methyl-isopropylketon, Methyl-isobutylketon, Acetonitril, Propionitril, N,N-Dimethyl-formamid, N,N-Dimethyl-acetamid und N-Methyl-pyrrolidon besonders bevorzugt eingesetzt.

Die Reaktionstemperaturen können bei der Durchführung des erfindungsgemäßen Verfahrens in einem größeren Bereich variiert werden. Im allgemeinen arbeitet man bei Temperaturen zwischen 20°C und 150°C, vorzugsweise bei Temperaturen zwischen 20°C und 120°C, insbesondere bei Temperaturen zwischen 20°C und 100°C.

Das erfindungsgemäße Verfahren wird im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Zur Durchführung des erfindungsgemäßen Verfahrens zur Herstellung der Verbindungen der Formel (I) setzt man pro Mol an Triazolinon der Formel (II) im allgemeinen 0,9 bis 1,5 Mol, vorzugsweise 1,0 bis 1,2 Mol an Sulfonsäurehalogenid der Formel (III) und 1,0 bis 3,0 Mol, vorzugsweise 1,5 bis 2,5 Mol Metallcyanat der Formel (IV) ein.

In einer bevorzugten Ausführungsform des erfindunggemäßen Verfahrens wird das Triazolinon der Formel (II) in einem geeigneten Verdünnungsmittel vorgelegt und das Metallcyanat der Formel (IV) und das Sulfonsäurechlorid der Formel (III) werden nacheinander dazu gegeben. Dann wird die Mischung bis zum Ende der Umsetzung bei der erforderlichen Temperatur gerührt.

Die Aufarbeitung kann nach üblichen Methoden durchgeführt werden. Beispielsweise wird nach Abkühlen unter vermindertem Druck eingeengt, der Rückstand mit einer wässrigen Säure, wie z.B. Salzsäure, angesäuert und dann das Reaktionsprodukt mit einem mit Wasser praktisch nicht mischbaren organischen Lösungsmittel, wie z.B. Methylenchlorid extrahiert. Die organische Extraktionslösung wird getrocknet und filtriert. Das Filtrat wird eingeengt, der Rückstand (Rohprodukt) gegebenenfalls durch Digerieren mit einem geeigneten Lösungsmittel, wie z.B. Diethylether zur Kristallisation gebracht und das kristalline Produkt durch Absaugen isoliert.

Die nach dem erfindungsgemäßen Verfahren herzustellenden Sulfonylaminocarbonyltriazolinone der Formel (I) können als Herbizide verwendet werden (vgl. EP-A 341489, EP-A 422469, EP-A 425948, EP-A 431291, EP-A 507171, EP-A 534266).

### Herstellungsbeispiele:

### Beispiel 1

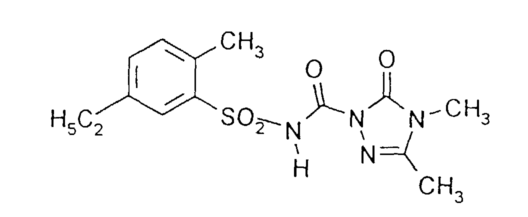

1,7 g (15 mMol) 4,5-Dimethyl-2,4-dihydro-3H-1,2,4-triazol-3-on werden mit 100 ml Acetonitril verrührt. Zu dieser Mischung werden nacheinander 2,0 g (30 mMol) Natriumcyanat und 3,3 g (15 mMol) 2-Methyl-5-ethyl-benzolsulfonsäurechlorid gegeben und die Reaktionsmischung wird unter Rühren 18 Stunden unter Rückfluß erhitzt. Nach Abkühlen auf Raumtemperatur (ca. 20°C) wird im Wasserstrahlvakuum eingeengt, der verbleibende Rückstand mit 2 ml konz. Salzsäure versetzt und mit Methylenchlorid extrahiert. Die organische Extraktionslösung wird mit Magnesiumsulfat getrocknet und filtriert. Vom Filtrat wird das Lösungsmittel im Wasserstrahlvakuum sorgfältig abdestilliert, der Rückstand durch Digerieren mit Diethylether zur Kristallisation gebracht und das kristalline Produkt durch Absaugen isoliert.

Man erhält 3,4 g (68% der Theorie) 4,5-Dimethyl-2-(2-methyl-5-ethyl-phenylsulfonyl-amino-carbonyl)-2,4-dihydro-3H-1,2,4-triazol-3-on vom Schmelzpunkt 145°C.

## Patentansprüche

1. Verfahren zur Herstellung von Sulfonylaminocarbonyltriazolinonen der allgemeinen Formel (I), 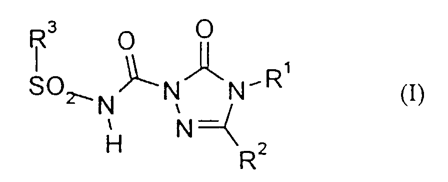 in welcher
R¹ für einen gegebenenfalls substituierten Rest aus der Reihe Alkyl, Alkenyl, Alkinyl, Alkoxy, Alkenyloxy, Dialkylamino, Cycloalkyl, Cycloalkyloxy, Cycloalkylalkyl, Aryl, Arylalkyl steht,
R² für Wasserstoff, Halogen oder für einen gegebenenfalls substituierten Rest aus der Reihe Alkyl, Alkenyl, Alkinyl, Alkoxy, Alkenyloxy, Alkinyloxy, Alkylthio, Alkenylthio, Alkinylthio, Alkylsulfinyl, Alkylsulfonyl, Dialkylamino, Cycloalkyl, Cycloalkenyl, Cycloalkyloxy, Cycloalkenyloxy, Cycloalkylthio, Cycloalkenylthio, Cycloalkylalkyl, Cycloalkenylalkyl, Aryl, Aryloxy, Arylthio, Arylalkyl, Arylalkyloxy, Arylalkylthio steht, und
R³ für einen gegebenenfalls substituierten Rest aus der Reihe Alkyl, Cycloalkyl, Cycloalkylalkyl, Aryl, Arylalkyl, Heterocyclyl, Heterocyclylalkyl steht,
dadurch gekennzeichnet, daß man Triazolinone der allgemeinen Formel (II), 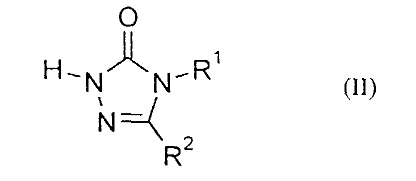 in welcher
R¹ und R² die oben angegebene Bedeutung haben,
mit Sulfonsäurehalogeniden der allgemeinen Formel (III),
R³-SO₂-X (III)
in welcher
R³ die oben angegebene Bedeutung hat und
X für Halogen steht,
und Metallcyanaten der allgemeinen Formel (IV),
MOCN (IV)
in welcher
M für ein Alkalimetall oder ein Erdalkalimetall-äquivalent steht,
gegebenenfalls in Gegenwart eines Reaktionshilfsmittels und gegebenenfalls in Gegenwart eines Verdünnungsmittels bei Temperaturen zwischen 20°C und 150°C umsetzt.

2. Verfahren nach Anspruch 1 zur Herstellung von Sulfonylaminocarbonyltriazolinon der Formel (I), in welcher
R¹ für gegebenenfalls durch Halogen, Cyano, C₁-C₄-Alkoxy, C₁-C₄-Alkyl-carbonyl oder C₁-C₄-Alkoxy-carbonyl substituiertes Alkyl mit 1 bis 6 Kohlenstoffatomen, für jeweils gegebenenfalls durch Halogen substituiertes Alkenyl oder Alkinyl mit jeweils 2 bis 6 Kohlenstoffatomen, für jeweils gegebenenfalls durch Halogen substituiertes Alkoxy, Alkenyloxy oder Dialkylamino mit jeweils bis zu 5 Kohlenstoffatomen in den Alkyl- bzw. Alkenyl-gruppen, für jeweils gegebenenfalls durch Halogen, Cyano und/oder C₁-C₄-Alkyl substituiertes Cycloalkyl, Cycloalkyloxy oder Cycloalkylalkyl mit jeweils 3 bis 6 Kohlenstoffatomen in den Cycloalkylgruppen und gegebenenfalls 1 bis 4 Kohlenstoffatomen im Alkylteil, oder für jeweils gegebenenfalls durch Halogen, Cyano, Nitro, C₁-C₄-Alkyl, C₁-C₄-Halogenalkyl, C₁-C₄-Alkoxy und/oder C₁-C₄-Halogenalkoxy substituiertes Phenyl, Naphthyl, Phenylalkyl oder Naphthylalkyl mit 1 bis 4 Kohlenstoffatomen in den Alkylteilen steht,
R² für Wasserstoff, Halogen, für gegebenenfalls durch Halogen, Cyano, C₁-C₄-Alkoxy, C₁-C₄-Alkyl-carbonyl oder C₁-C₄-Alkoxy-carbonyl substituiertes Alkyl mit 1 bis 6 Kohlenstoffatomen, für jeweils gegebenenfalls durch Halogen substituiertes Alkenyl oder Alkinyl mit jeweils 2 bis 6 Kohlenstoffatomen, für jeweils gegebenenfalls durch Halogen substituiertes Alkoxy, Alkenyloxy, Alkinyloxy, Alkylthio, Alkenylthio, Alkinylthio, Alkylsulfinyl, Alkylsulfonyl, Dialkylamino mit jeweils bis zu 5 Kohlenstoffatomen, für jeweils gegebenenfalls durch Halogen, Cyano oder C₁-C₄-Alkyl substituiertes Cycloalkyl, Cycloalkenyl, Cycloalkyloxy, Cycloalkenyloxy, Cycloalkylthio, Cycloalkenylthio, Cycloalkylalkyl oder Cycloalkenylalkyl mit jeweils 3 bis 6 Kohlenstoffatomen in den Cycloalkylgruppen und gegebenenfalls 1 bis 4 Kohlenstoffatomen in den Alkylgruppen, für jeweils gegebenenfalls durch Halogen, Cyano, Nitro, C₁-C₄-Alkyl, C₁-C₄-Halogenalkyl, C₁-C₄-Alkoxy und/oder C₁-C₄-Halogenalkoxy substituiertes Phenyl, Naphthyl, Phenoxy, Naphthyloxy, Phenylthio, Naphthylthio, Phenylmethyl, Phenylethyl, Phenylmethoxy, Naphthylmethoxy, Phenylethoxy, Naphthylethoxy, Phenylmethylthio, Naphthylmethylthio, Phenylethylthio oder Naphthylethylthio steht, und
R³ für gegebenenfalls durch Halogen, Cyano, C₁-C₄-Alkoxy, C₁-C₄-Alkyl-carbonyl oder C₁-C₄-Alkoxy-carbonyl substituiertes Alkyl mit 1 bis 6 Kohlenstoffatomen, für jeweils gegebenenfalls durch Halogen, Cyano, Carboxy, C₁-C₄-Alkyl und/oder C₁-C₄-Alkoxy-carbonyl substituiertes Cycloalkyl oder Cycloalkylalkyl mit 3 bis 6 Kohlenstoffatomen in den Cycloalkylgruppen und gegebenenfalls 1 bis 4 Kohlenstoffatomen im Alkylteil, oder für jeweils gegebenenfalls durch Halogen, Cyano, Carboxy, Nitro, C₁-C₄-Alkyl, C₁-C₄-Halogenalkyl, C₁-C₄-Alkoxy-C₁-C₄-alkyl, C₁-C₄-Alkoxy, C₁-C₄-Halogenalkoxy, C₁-C₄-Alkoxy-C₁-C₄-alkoxy, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl, Di-(C₁-C₃-alkyl)-amino-sulfonyl, N-(C₁-C₃-Alkoxy)-N-(C₁-C₃-alkyl)-amino-sulfonyl, C₁-C₄-Alkyl-carbonyl, C₁-C₄-Alkoxy-carbonyl, C₁-C₄-Halogenalkoxy-carbonyl, Di-(C₁-C₃-alkyl)-amino-carbonyl, C₁-C₂-Alkoxy-C₁-C₂-alkoxy-carbonyl, C₃-C₆-Cycloalkyl, C₃-C₆-Cycloalkyl-methyl, C₃-C₆-Cycloalkyl-carbonyl, Phenyl oder Phenoxy substituiertes Phenyl, Naphthyl, Benzyl, Phenylethyl, Pyridyl, Pyridylmethyl, Pyridylethyl, Chinolyl, Chinolylmethyl, Thienyl, Thienylmethyl, Pyrazolyl oder Pyrazolylmethyl steht.

3. Verfahren nach Anspruch 1, dadurch gekennzeichnet, daß man bei Temperaturen zwischen 20°C und 120°C arbeitet.

4. Verfahren nach Anspruch 1, dadurch gekennzeichnet, daß man als Metallcyanate der Formel (IV) Natrium-, Kalium-, Magnesium- oder Calciumcyanat einsetzt.

5. Verfahren nach Anspruch 1, dadurch gekennzeichnet, daß man als Reaktionshilfsmittel eine organische Stickstoffbase einsetzt.

6. Verfahren nach Anspruch 1, dadurch gekennzeichnet, daß man als Verdünnungsmittel ein inertes organisches Lösungsmittel einsetzt.

## Claims

1. Process for preparing sulphonylaminocarbonyltriazolinones of the general formula (I), 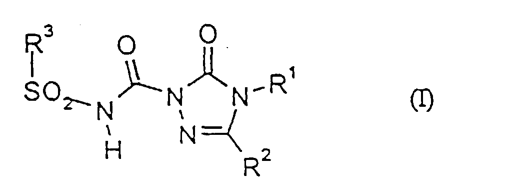 in which
R¹ represents an optionally substituted radical from the group comprising alkyl, alkenyl, alkinyl, alkoxy, alkenyloxy, dialkylamino, cycloalkyl, cycloalkyloxy, cycloalkylalkyl, aryl or arylalkyl,
R² represents hydrogen, halogen, or represents an optionally substituted radical from the group comprising alkyl, alkenyl, alkinyl, alkoxy, alkenyloxy, alkinyloxy, alkylthio, alkenylthio, alkinylthio, alkylsulphinyl, alkylsulphonyl, dialkylamino, cycloalkyl, cycloalkenyl, cycloalkyloxy, cycloalkenyloxy, cycloalkylthio, cycloalkenylthio, cycloalkylalkyl, cycloalkenylalkyl, aryl, aryloxy, arylthio, arylalkyl, arylalkyloxy or arylalkylthio, and
R³ represents an optionally substituted radical from the group comprising alkyl, cycloalkyl, cycloalkylalkyl, aryl, arylalkyl, heterocyclyl or heterocyclylalkyl,
characterized in that triazolinones of the general formula (II), 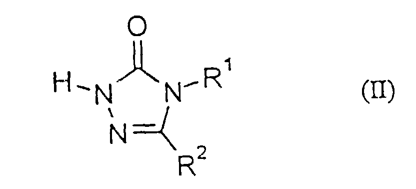 in which
R¹ and R² have the abovementioned meaning,
are reacted with sulphonyl halides of the general formula (III)
R³-SO₂-X (III)
in which
R³ has the abovementioned meaning, and
X represents halogen,
and metal cyanates of the general formula (IV)
MOCN (IV)
in which
M represents an alkali metal or one alkaline earth metal equivalent,
optionally in the presence of a reaction auxiliary and optionally in the presence of a diluent at temperatures of between 20°C and 150°C.

2. Process according to Claim 1 for preparing sulphonylaminocarbonyltriazolinones of the formula (I), in which
R¹ represents alkyl which has from 1 to 6 carbon atoms and which is optionally substituted by halogen, cyano, C₁-C₄-alkoxy, C₁-C₄-alkyl-carbonyl or C₁-C₄-alkoxy-carbonyl, represents alkenyl or alkinyl which have in each case from 2 to 6 carbon atoms and which are in each case optionally substituted by halogen, represents alkoxy, alkenyloxy or dialkylamino which have in each case up to 5 carbon atoms in the alkyl or alkenyl groups and which are in each case optionally substituted by halogen, represents cycloalkyl, cycloalkyloxy, or cycloalkylalkyl which have in each case from 3 to 6 carbon atoms in the cycloalkyl groups and optionally 1 to 4 carbon atoms in the alkyl moiety and which are in each case optionally substituted by halogen, cyano or C₁-C₄-alkyl, or represents phenyl, naphthyl, phenylalkyl or naphthylalkyl which have from 1 to 4 carbon atoms in the alkyl moieties and which are in each case optionally substituted by halogen, cyano, nitro, C₁-C₄-alkyl, C₁-C₄-halogenoalkyl, C₁-C₄-alkoxy and/or C₁-C₄-halogenoalkoxy,
R² represents hydrogen, or halogen, represents alkyl which have from 1 to 6 carbon atoms and which is optionally substituted by halogen, cyano, C₁-C₄-alkoxy, C₁-C₄-alkylcarbonyl or C₁-C₄-alkoxycarbonyl, represents alkenyl or alkinyl which have in each case from 2 to 6 carbon atoms and which are in each case optionally substituted by halogen, represents alkoxy, alkenyloxy, alkinyloxy, alkylthio, alkenylthio, alkinylthio, alkylsulphinyl, alkylsulphonyl or dialkylamino which have in each case up to 5 carbon atoms and which are in each case optionally substituted by halogen, cyano or C₁-C₄-alkyl, represents cycloalkyl, cycloalkenyl, cycloalkyloxy, cycloalkenyloxy, cycloalkylthio, cycloalkenylthio, cycloalkylalkyl or cycloalkenylalkyl which have in each case from 3 to 6 carbon atoms in the cycloalkyl groups and optionally from 1 to 4 carbon atoms in the alkyl groups and which are optionally substituted by halogen, cyano or C₁-C₄-alkyl, or represents phenyl, naphthyl, phenoxy, naphthyloxy, phenylthio, naphthylthio, phenylmethyl, phenylethyl, phenylmethoxy, naphthylmethoxy, phenylethoxy, naphthylethoxy, phenylmethylthio, naphthylmethylthio, phenylethylthio or naphthylethylthio, which are in each case optionally substituted by halogen, cyano, nitro, C₁-C₄-alkyl, C₁-C₄-halogenoalkyl, C₁-C₄-alkoxy and/or C₁-C₄-halogenoalkoxy, and
R³ represents alkyl which has from 1 to 6 carbon atoms and which is optionally substituted by halogen, cyano, C₁-C₄-alkoxy, C₁-C₄-alkylcarbonyl or C₁-C₄-alkoxycarbonyl, represents cycloalkyl or cycloalkylalkyl which have from 3 to 6 carbon atoms in the cycloalkyl groups and optionally from 1 to 4 carbon atoms in the alkyl moiety and which are in each case optionally substituted by halogen, cyano, carboxyl, C₁-C₄-alkyl and/or C₁-C₄-alkoxycarbonyl, or represents phenyl, naphthyl, benzyl, phenylethyl, pyridyl, phenylmethyl, pyridylethyl, quinolyl, quinolylmethyl, thienyl, thienylmethyl, pyrazolyl or pyrazolylmethyl which are in each case optionally substituted by halogen, cyano, carboxyl, nitro, C₁-C₄-alkyl, C₁-C₄-halogenoalkyl, C₁-C₄-alkoxy-C₁-C₄-alkyl, C₁-C₄-alkoxy, C₁-C₄-halogenoalkoxy, C₁-C₄-alkoxy-C₁-C₄-alkoxy, C₁-C₄-alkylthio, C₁-C₄-halogenoalkylthio, C₁-C₄-alkylsulphinyl, C₁-C₄-halogenoalkylsulphinyl, C₁-C₄-alkylsulphonyl, C₁-C₄-halogenoalkylsulphonyl, di-(C₁-C₃-alkyl)amino-sulphonyl, N-(C₁-C₃-alkoxy)-N-(C₁-C₃-alkyl)-aminosulphonyl, C₁-C₄-alkylcarbonyl, C₁C₄-alkoxycarbonyl, C₁-C₄-halogenoalkoxycarbonyl, di-(C₁-C₃-alkyl)-aminocarbonyl, C₁-C₂-alkoxy-C₁-C₂-alkoxycarbonyl, C₃-C₆-cycloalkyl, C₃-C₆-cycloalkylmethyl, C₃-C₆-cycloalkylcarbonyl, phenyl or phenoxy.

3. Process according to Claim 1, characterized in that temperatures of between 20°C and 120°C are employed.

4. Process according to Claim 1, characterized in that sodium cyanate, potassium cyanate, magnesium cyanate or calcium cyanate are employed as metal cyanates of the formula (IV).

5. Process according to Claim 1, characterized in that an organic nitrogen base is employed as reaction auxiliary.

6. Process according to Claim 1, characterized in that an inert organic solvent is employed as diluent.

## Revendications

1. Procédé pour la préparation de sulfonylaminocarbonyltriazolinones répondant à la formule générale (I) 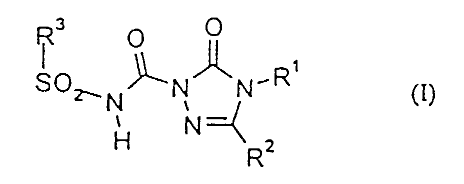 dans laquelle
R¹ représente un radical éventuellement substitué choisi parmi la série comprenant un radical alkyle, un radical alcényle, un radical alcynyle, un radical alcoxy, un radical alcényloxy, un radical dialkylamino, un radical cycloalkyle, un radical cycloalkyloxy, un radical cycloalkylalkyle, un radical aryle, un radical arylalkyle,
R² représente un atome d'hydrogène, un atome d'halogène ou encore un radical éventuellement substitué choisi parmi la série comprenant un radical alkyle, un radical alcényle, un radical alcynyle, un radical alcoxy, un radical alcényloxy, un radical alcynyloxy, un radical alkylthio, un radical alcénylthio, un radical alcynylthio, un radical alkylsulfinyle, un radical alkylsulfonyle, un radical dialkylamino, un radical cycloalkyle, un radical cycloalcényle, un radical cycloalcoxy, un radical cycloalcényloxy, un radical cycloalkylthio, un radical cycloalcénylthio, un radical cycloalkylalkyle, un radical cycloalcénylalkyle, un radical aryle, un radical aryloxy, un radical arylthio, un radical arylalkyle, un radical arylalcoxy, un radical arylalkylthio, et
R³ représente un radical éventuellement substitué choisi parmi la série comprenant un radical alkyle, un radical cycloalkyle, un radical cycloalkylalkyle, un radical aryle, un radical arylalkyle, un radical hétérocyclyle, un radical hétérocyclylalkyle,
caractérisé en ce qu'on fait réagir des triazolinones répondant à la formule générale (II) 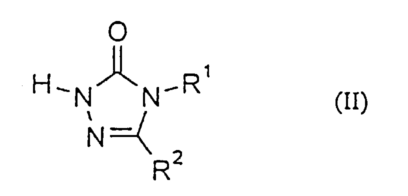 dans laquelle
R¹ et R² ont la signification indiquée ci-dessus,
avec des halogénures d'acides sulfoniques répondant à la formule générale (III)
R³-SO₂-X (III)
dans laquelle
R³ a la signification indiquée ci-dessus et
X représente un atome d'halogène,
et avec des cyanates métalliques répondant à la formule générale (IV)
MOCN (IV)
dans laquelle
M représente un métal alcalin ou un équivalent de métal alcalino-terreux,
éventuellement en présence d'un adjuvant réactionnel et éventuellement en présence d'un diluant, à des températures entre 20°C et 150°C.

2. Procédé selon la revendication 1, pour la préparation de la sulfonylaminocarbonyltriazolinone de formule (I) dans laquelle
R¹ représente un groupe alkyle contenant de 1 à 6 atomes de carbone possédant le cas échéant un ou plusieurs substituants identiques ou différents halogéno, cyano, alcoxy en C₁-C₄, alkyl(en C₁-C₄)carbonyle ou alcoxy(en C₁-C₄)carbonyle; un groupe alcényle ou un groupe alcynyle contenant respectivement de 2 à 6 atomes de carbone, chacun de ces groupes portant le cas échéant un ou plusieurs substituants halogéno identiques ou différents; un groupe alcoxy, un groupe alcényloxy ou un groupe dialkylamino, dont les groupes alkyle, respectivement alcényle contiennent jusqu'à 5 atomes de carbone, chacun de ces groupes portant le cas échéant un ou plusieurs substituants halogéno identiques ou différents; un groupe cycloalkyle, un groupe cycloalcoxy ou un groupe cycloalkylalkyle dont les groupes cycloalkyle portent respectivement de 3 à 6 atomes de carbone et dont la fraction alkyle porte le cas échéant de 1 à 4 atomes de carbone, chacun de ces groupes portant éventuellement un ou plusieurs substituants identiques ou différents halogéno, cyano et/ou alkyle en C₁-C₄; ou encore un groupe phényle, un groupe naphtyle, un groupe phénylalkyle ou un groupe naphtylalkyle dont les fractions alkyle portent de 1 à 4 atomes de carbone, chacun de ces groupes portant éventuellement un ou plusieurs substituants identiques ou différents halogéno, cyano, nitro, alkyle en C₁-C₄, halogénalkyle en C₁-C₄, alcoxy en C₁-C₄ et/ou halogénalcoxy en C₁-C₄,
R² représente un atome d'hydrogène; un atome d'halogène; un groupe alkyle contenant de 1 à 6 atomes de carbone portant éventuellement un ou plusieurs substituants identiques ou différents halogéno, cyano, alcoxy en C₁-C₄, alkyl(en C₁-C₄)carbonyle ou alcoxy(en C₁-C₄)carbonyle; un groupe alcényle ou un groupe alcynyle contenant respectivement de 2 à 6 atomes de carbone, chacun de ces groupes portant le cas échéant un ou plusieurs substituants halogéno identiques ou différents; un groupe alcoxy, un groupe alcényloxy, un groupe alcynyloxy, un groupe alkylthio, un groupe alcénylthio, un groupe alcynylthio, un groupe alkylsulfinyle, un groupe alkylsulfonyle, un groupe dialkylamino contenant respectivement jusqu'à 5 atomes de carbone, chacun de ces groupes portant le cas échéant un ou plusieurs substituants halogéno identiques ou différents; un groupe cycloalkyle, un groupe cycloalcényle, un groupe cycloalcoxy, un groupe cycloalcényloxy, un groupe cycloalkylthio, un groupe cycloalcénylthio, un groupe cycloalkylalkyle ou un groupe cycloalcénylalkyle dont les groupes cycloalkyle contiennent respectivement de 3 à 6 atomes de carbone et dont les groupes alkyle contiennent le cas échéant de 1 à 4 atomes de carbone, chacun de ces groupes portant éventuellement un ou plusieurs substituants identiques ou différents halogéno, cyano ou alkyle en C₁-C₄; un groupe phényle, un groupe naphtyle, un groupe phénoxy, un groupe naphtyloxy, un groupe phénylthio, un groupe naphtylthio, un groupe phénylméthyle, un groupe phényléthyle, un groupe phénylméthoxy, un groupe naphtylméthoxy, un groupe phényléthoxy, un groupe naphtyléthoxy, un groupe phénylméthylthio, un groupe naphtylméthylthio, un groupe phényléthylthio ou un groupe naphtyléthylthio, chacun de ces groupes portant éventuellement un ou plusieurs substituants identiques ou différents halogéno, cyano, nitro, alkyle en C₁-C₄, halogénalkyle en C₁-C₄, alcoxy en C₁-C₄ et/ou halogénalcoxy en C₁-C₄, et
R³ représente un groupe alkyle contenant de 1 à 6 atomes de carbone portant éventuellement un ou plusieurs substituants identiques ou différents halogéno, cyano, alcoxy en C₁-C₄, alkyl(en C₁-C₄)carbonyle ou alcoxy(en C₁-C₄)carbonyle; un groupe cycloalkyle ou un groupe cycloalkylalkyle dont les groupes cycloalkyle contiennent de 3 à 6 atomes de carbone et dont la fraction alkyle contient éventuellement de 1 à 4 atomes de carbone, chacun de ces groupes portant le cas échéant un ou plusieurs substituants identiques ou différents halogéno, cyano, carboxyle, alkyl(en C₁-C₄)- et/ou alcoxy(en C₁-C₄)-carbonyle; ou encore un groupe phényle, un groupe naphtyle, un groupe benzyle, un groupe phényléthyle, un groupe pyridyle, un groupe pyridylméthyle, un groupe pyridyléthyle, un groupe quinoléyle, un groupe quinoléylméthyle, un groupe thiényle, un groupe thiénylméthyle, un groupe pyrazolyle ou un groupe pyrazolylméthyle, chacun de ces groupes portant éventuellement un ou plusieurs substituants identiques ou différents halogéno, cyano, carboxyle, nitro, alkyle en C₁-C₄, halogénalkyle en C₁-C₄, alcoxy(en C₁-C₄)alkyle en C₁-C₄, alcoxy en C₁-C₄, halogénalcoxy en C₁-C₄, alcoxy(en C₁-C₄)alcoxy en C₁-C₄, alkyl(en C₁-C₄)thio, halogénalkyl(en C₁-C₄)thio, alkyl(en C₁-C₄)sulfinyle, halogénalkyl(en C₁-C₄)sulfinyle, alkyl(en C₁-C₄)sulfonyle, halogénalkyl(en C₁-C₄)sulfonyle, di-(alkyl en C₁-C₃)aminosulfonyle, N-(alcoxy en C₁-C₃)-N-(alkyl en C₁-C₃)aminosulfonyle, alkyl(en C₁-C₄)carbonyle, alcoxy(en C₁-C₄)-carbonyle, halogénalcoxy(en C₁-C₄)carbonyle, di-(alkyl en C₁-C₃)aminocarbonyle, alcoxy(en C₁-C₂)alcoxy(en C₁-C₂)carbonyle, cycloalkyle en C₃-C₆, cycloalkyl (en C₃-C₆)méthyle, cycloalkyl(en C₃-C₆)carbonyle, phényle ou phénoxy.

3. Procédé selon la revendication 1, caractérisé en ce qu'on travaille à des températures entre 20°C et 120°C.

4. Procédé selon la revendication 1, caractérisé en ce qu'on met en oeuvre, comme cyanates métalliques de formule (IV), le cyanate de sodium, le cyanate de potassium, le cyanate de magnésium ou le cyanate de calcium.

5. Procédé selon la revendication 1, caractérisé en ce qu'on met en oeuvre, comme adjuvant réactionnel, une base azotée organique.

6. Procédé selon la revendication 1, caractérisé en ce qu'on met en oeuvre, comme diluant, un solvant organique inerte.
